# EUROPEAN PATENT SPECIFICATION

(11) **EP 2 385 113 B1**
(45) Date of publication and mention of the grant of the patent: **07.05.2014**
(21) Application number: 11158518.8
(22) Date of filing: 16.03.2011
(51) Int. Cl.: C12N 9/64, C12Q 1/68

(54) **Genetic test for genetic disposition of haemophilia B in Rhodesian Ridgeback**
Gentest für die genetische Disposition von Hämophilie B im Rhodesian Ridgeback
Test génétique pour disposition génétique de l'hémophilie B chez le chien de Rhodésie

(30) Priority: 17.03.2010 EP 10156797
(43) Date of publication of application: 09.11.2011
(73) Proprietor: Laboklin GmbH & Co. KG, 97688 Bad Kissingen (DE)
(72) Inventor: Müller, Elisabeth, Dr., 97688 Bad Kissingen (DE); Kühnlein, Petra, Dr., 97456 Dittelbrunn (DE); Kehl, Alexandra, 97702 Wermerichshausen (DE)
(74) Representative: advotec.

(56) References cited:
- J. P. EVANS: "Canine Hemophilia B Resulting from a Point Mutation with Unusual Consequences", PROCEEDINGS OF THE NATIONAL ACADEMY OF SCIENCES, vol. 86, no. 24, 15 December 1989 (1989-12-15), pages 10095-10099, XP55004071, ISSN: 0027-8424, DOI: 10.1073/pnas.86.24.10095
- MAUSER A E ET AL: "A deletion mutation causes hemophilia B in Lhasa Apso dogs", BLOOD, vol. 88, no. 9, 1996, pages 3451-3455, XP002659954, ISSN: 0006-4971
- BOTTEMA C D K ET AL: "MISSENSE MUTATIONS AND EVOLUTIONARY CONSERVATION OF AMINO ACIDS: EVIDENCE THAT MANY OF THE AMINO ACIDS IN FACTOR IX FUNCTION AS SPACER ELEMENTS", AMERICAN JOURNAL OF HUMAN GENETICS, AMERICAN SOCIETY OF HUMAN GENETICS, CHICAGO, IL, US, vol. 49, no. 4, 1 January 1991 (1991-01-01), pages 820-838, XP009007557, ISSN: 0002-9297
- LUTZE G ET AL: "Hämophilie A und B beim Hund / Haemophilia A and B in dogs", LABORATORIUMS MEDIZIN / JOURNAL OF LABORATORY MEDICINE, WALTER DE GRUYTER GMBH & CO. KG, KIRCHHEIM, DE, vol. 24, no. 6-7, 1 January 2000 (2000-01-01), pages 319-324, XP009150975, ISSN: 0342-3026
- GU WEIKUAN ET AL: "Two distinct mutations cause severe hemophilia B in two unrelated canine pedigrees", THROMBOSIS AND HAEMOSTASIS, SCHATTAUER GMBH, DE; US, vol. 82, no. 4, 1 October 1999 (1999-10-01), pages 1270-1275, XP009150992, ISSN: 0340-6245
- GIANNELLI F ET AL: "Haemophilia B: Database of point mutations and short additions and deletions-fourth edition, 1993", NUCLEIC ACIDS RESEARCH, vol. 21, no. 13, 1993, pages 3075-3087, XP002659955, ISSN: 0305-1048
- MISCHKE R ET AL: "G244E in the canine factor IX gene leads to severe haemophilia B in Rhodesian Ridgebacks", VETERINARY JOURNAL, BAILLIERE TINDALL, LONDON, GB, vol. 187, no. 1, 1 January 2011 (2011-01-01), pages 113-118, XP027570503, ISSN: 1090-0233 [retrieved on 2010-12-25]
- F GIANNELLI ET AL: 'Haemophilia B (sixth edition): a database of point mutations and short additions and deletions' NUCLEIC ACIDS RESEARCH vol. 24, no. 1, 01 January 1996, pages 103 - 118, XP055036054 DOI: 10.1093/nar/24.1.103 ISSN: 0305-1048

## Description

The invention relates to a genetic test for genetic disposition of haemophilia B in Rhodesian Ridgeback by genotyping the canine factor IX gene.

### Introduction

Haemophilia B is a condition affecting the blood clotting process by lack of biologically active coagulation factor IX. Factor IX is a 56 kDa plasma glycoprotein that is required for activation of Factor X in the coagulation cascade. Canine haemophilia B represents a naturally occurring hereditary coagulopathy with a range in severity of clinical signs and coagulant activity in different breed variants. It has been reported in many breeds. In Rhodesian Ridgebacks, a severe form of haemophilia B with pronounced clinical signs was first described in Lutze et al. (2000). Currently it is the clinically most important canine haemophilia form in Germany.

BOTTEMA C D K et al.: "Missense mutations and evolutionary conservation of amino acids: evidence that many of the amino acids in factor IX function as spacer elements", American Journal of Human Genetics, American Society of Human Genetics, Chicago, IL, US, vol. 49. no. 4, 1 January 1991 (1991-01-01), pages 820-838, XP009007557, ISSN: 0002-9297. BOTTEMA C D K et al. discloses that in human factor IX missense mutations in factor IX-specific residues (i.e., those conserved in human, cow, dog, and mouse factor IX but not in the related proteases) are sixfold less likely to cause disease. Position 244 of dog factor IX, corresponds to position 207 in human factor IX and is a conserved G residue. Said document also refers to this position in humans as a missense mutation reported.

GIANNELLI F et al.: "Haemophilia B: Database of point mutations and short additions and deletions-fourth edition, 1993", Nucleic Acids Research, vol. 21, no. 13, 1993, pages 3075-3087, XP002659955, ISSN: 0305-1048. GIANNELLI F et al. discloses several mutations of human Factor IX causing haemophilia. One of them is a mutation G->A in the nucleotide, resulting in an amino acid chance at position 253, G253->E; this corresponds to the position 244 in canine (dog) factor IX, where the same mutation identified in the application is located.

F GIANNELLI et al.: "Haemophilia B (sixth edition): a database of point mutations and short additions and deletions", Nucleic Acids Research, vol. 24, no. 1, 1 January 1996 (1996-01-01), pages 103-118, XP55036054, ISSN: 0305-1048, DOI: 10.1093/nar/24.1.103. F GIANNELLI et al. further discloses a long list of mutations in human factor IX three of them at position 207, namely: G207->R , G207->stop and G207->E.

Different mutations in the gene encoding factor IX have been identified in different dog breeds, including Labrador, Lhasa Apso, Pit Bull Terrier, and Airedale Terrier resulting in clinical symptoms typical for haemophilia B (Evans et al., 1989; Mauser et al., 1996; Brooks et al., 1997; Gu et al., 1999; Table 11). To the author's knowledge, the genetic background of haemophilia B in Rhodesian Ridgebacks is unknown. Therefore the aim of this study was to investigate whether a genetic defect could be identified as underlying cause of haemophilia B in Rhodesian Ridgeback dogs. A genetic test would help to diagnose diseased dogs and identify genetically affected and carrier dogs.

It is the object of the present invention to provide a genetic test for the analysis of a genotype of a dog, preferably a Rhodesian Ridgeback in respect of haemophilia B.

This aim is achieved by the inventions as claimed in the independent claims. Advantageous embodiments are described in the dependent claims.

The problem is solved by providing a Canine factor IX gene according to Seq-ID No. 1 having a point mutation, wherein nucleotide 752 is A instead of G.

It has been found that a single point mutation in the canine factor IX leads to a very strong reduction in the activity of the factor and could be assigned to haemophilia B in the dog, preferably the Rhodesian Ridgebacks.

In Seq-ID No. 1 the nucleotide in position 752 is mutated from G to A. This mutation results in a glycine (GGA) to glutamic acid (GAA) exchange in position 244 in the catalytic domain of the haemophilic factor IX (Seq-ID No. 3; wild type Seq-ID No. 2).

The invention therefore relates to a genetic test for identification of at least one point mutation in Seq-ID No. 1 of the genome of the dog, wherein at least one point mutation leads to an amino acid exchange in position 244 of canine factor IX.

The point mutation is a point mutation in nucleotide 752 of Seq-ID No. 1, in which nucleotide 752 is A instead of G.

The analysis may also be used to detect further mutations in sequence Seq-ID No. 1, for example mutations which are not related to haemophilia B.

In an embodiment of the invention the method for the analysis of a genotype of a dog in respect of haemophilia B comprises the identification of a point mutation in Seq-ID No. 1 of the genome of the dog, wherein one point mutation is a point mutation in which nucleotide 752 is A instead of G.

By this test it is possible to identify the genotype of the Rhodesian Ridgeback in respect of haemophilia B, and especially if the dog is a heterozygous carrier of the mutation.

The procedures to detect a point mutation in a genome are known to the person skilled in the art. Possible methods are sequencing the region of the genome, where the mutation is localised, with specific primers and then comparing the sequence with the wild type sequence. The sequencing may be done directly with the genomic DNA or by amplification of the selected region and then sequencing the amplified sequence.

Other possible methods comprise ligase chain reaction (LCR), restriction fragment length polymorphism (RFLP), mass spectroscopy (MALDI-TOF), SSCP, DGGE, pyrosequencing, dHPLC, allele-specific hybridization, molecular beacons, microarrays, Flap endonuclease (FEN), real-time PCR, allele specific PCR, Taqman assay.

For example PCR may be used for the amplification of the region of the point mutation, the region of Seq-ID No. 1, which contains at least one of the point mutation in Seq-ID No. 1 of the genome of the dog, wherein nucleotide 752 is A instead of G.

In a preferred embodiment the dog is a Rhodesian Ridgeback.

Another embodiment of the invention is a process for the determination of the genetic disposition for haemophilia B of a dog by the use of the canine factor IX gene of the dog according to Seq-ID No. 1 having at least one point mutation in Seq-ID No. 1 of the genome of the dog, wherein at least one point mutation leads to an amino acid exchange in position 244 of canine factor IX.

The point mutation is a point mutation on nucleotide 752, in which this nucleotide is A instead of G.

In a preferred embodiment the dog is a Rhodesian Ridgeback.

Another embodiment of the invention this process may be used for the selection of a Rhodesian Ridgeback for breeding.

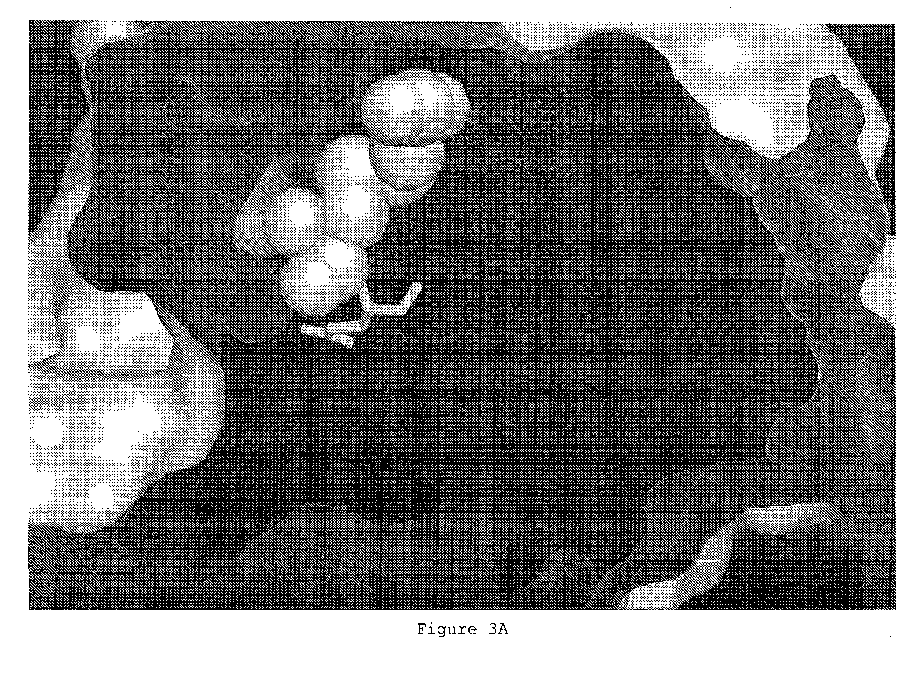

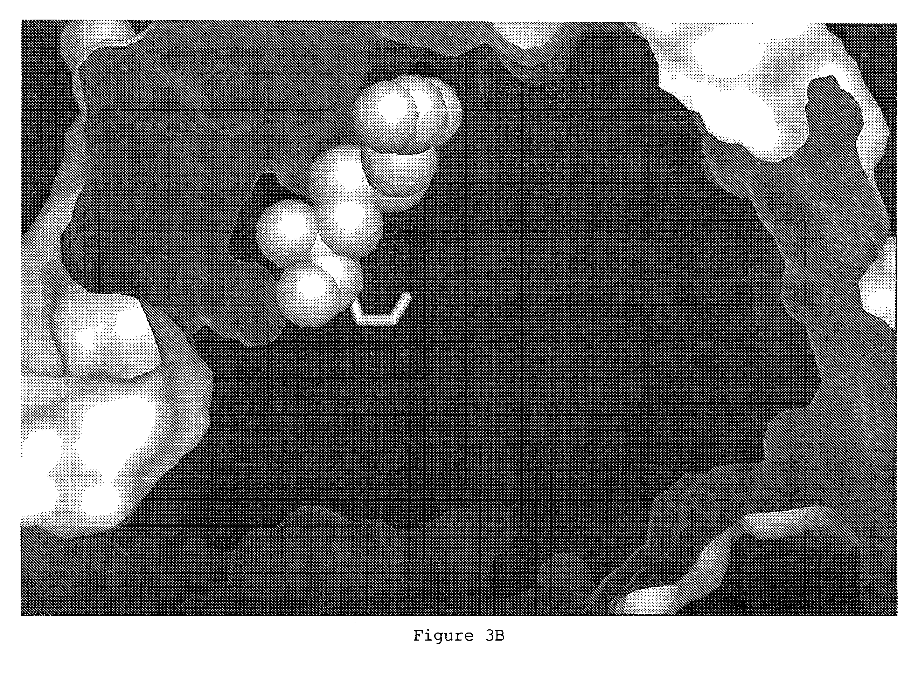

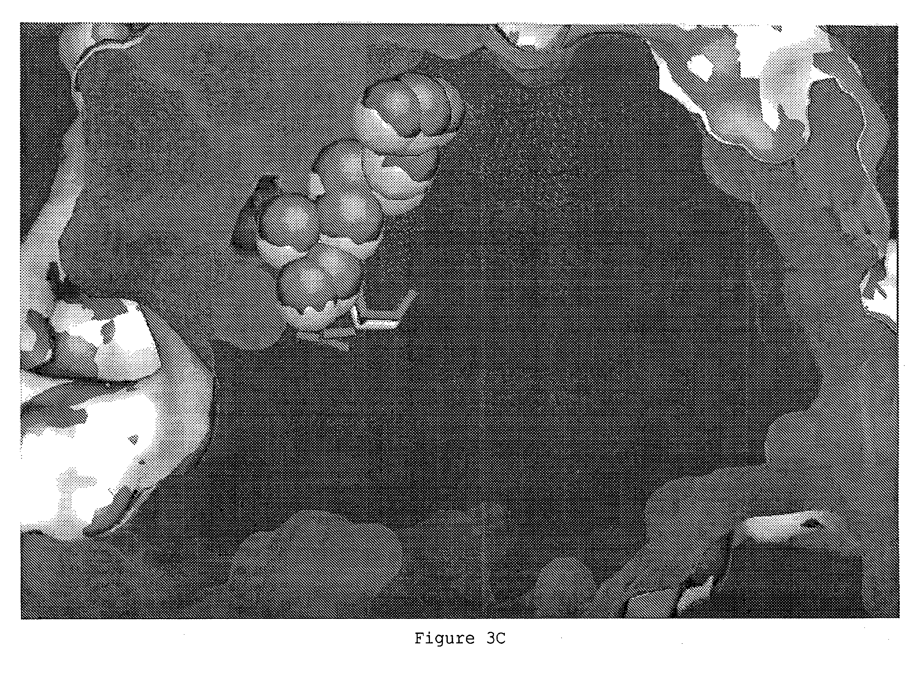

Even if no multiple back-referenced claims are drawn, all reasonable combinations of the features in the claims shall be disclosed.
- Table 1: Literature survey of mutations on canine factor IX gene in dogs with haemophilia;
- Table 2: Results of sequence analysis of the canine factor IX DNA and TaqMan genotyping assay for a G-A missense mutation in exon 7 in Rhodesian Ridgebacks dogs with different status of haemophilia B (Signalement, pedigree: Confirmed carrier, mother of Nos. 4 and 5; Clinical signs: Severe bleeding after minor surgeries; Sequence analysis: n= Normal c=carrier a=affected) and injuries, occasional spontaneous bleeding (+/-));
- Fig. 1: Amino acid sequence alignment of protease domain of canine factor IX (CFIX) with humane factor IX (HFIX), bovine trypsin (TRYP), porcine elastase (ELAS), bovine chymotrypsin (CHYM) and porcine kallikrein (KALL);
- Fig. 2: Factor IX domain composition: detected features according to ProSite and SMART. Key domains of factor IX include N-terminal gamma-carboxyglutamate (GLA, see grey arrow) containing domain (hexagon), the calcium binding EGF domain (pentagon, disulfide bonds indicated in grey) and trypsin-like serine protease domain (ellipse, C-terminal). The three active sites residues (H258, D306 and S402) of this domain are shown as small diamonds as well as the disulfide bonds (grey rectangular lines). A detailed list of the features of this domain and positions is shown in Table 3;
- Fig. 3: Figure 3A: Protein mutated inside view: disulfide (spheres), active site and mutates amino acid (ribbon);
Comparison of structure of mutated canine factor IX and wild type factor IX. Illustration of the affected residues of factor IX from the inside; Figure 3A is wild type; Figure 3B is mutated protein. Spherical shape: disulfide bridge; ribbon shape: mutated amino acid. The view includes the active site; Figure 3C shows an Overlay of Figures 3A and 3B (dark gray regions: Figure 3A; light grey: Figure 3B); Note how change of residue 244 leads to a change in overall protein geometry. Especially the disulfide bridge is affected.

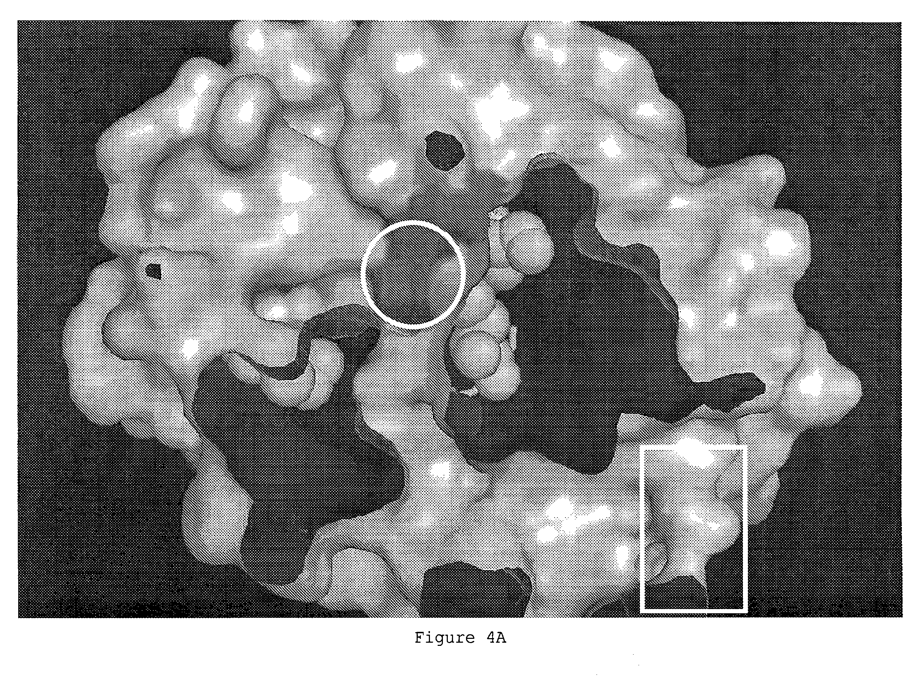

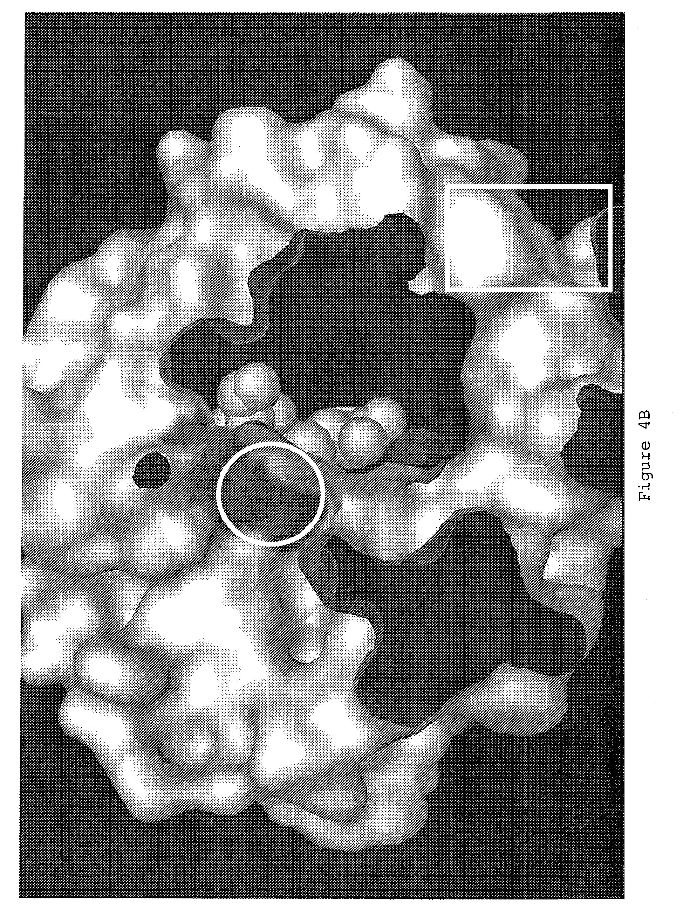

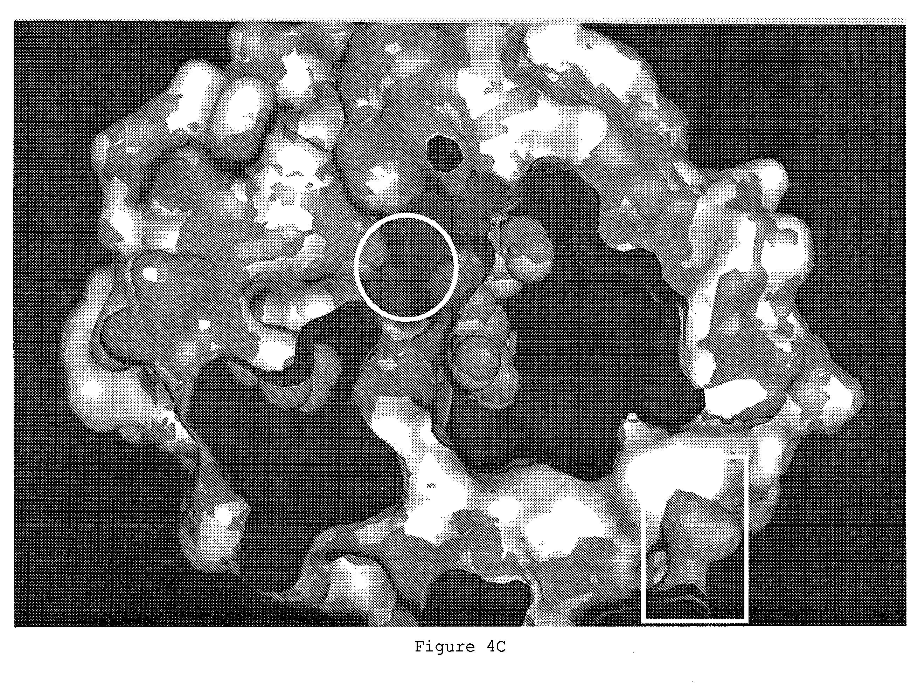

- Fig. 4: Model of the affected residues of the factor IX molecules from the outside; Figure 4A shows the mutated protein; Figure 4B the wild type; Spherical shape: disulfide bridge; ribbon shape: mutated amino acid; dark surface around the white circle: active site; clearly visible are the disulfide bridges and the active sites. It is clearly visible, that the mutated amino acid causes a shift in the spatial structure in the disulfide bridge and thus also in the active site (white circle; dark grey). It also causes a shift in the periphery of structures (lower right corner, white rectangle) with the effect, that an additional bond is forming a loop. Both can also be seen in the overlay of figure 4A and figure 4B shown in figure 4C (dark gray: figure 4A; light gray figure 4B).

### DETAILED DESCRIPTION OF THE INVENTION

### Materials and methods

### Study design

The study included six phenotypically affected male dogs, four suspected carriers, and 12 healthy dogs. From all dogs with known or suspected haemophilia B status, factor IX activity was measured to verify the status for haemophilia B. From all these dogs, DNA was isolated from EDTA blood, and the coding region of canine factor IX was amplified and sequenced. The obtained DNA sequence was compared with the wild type canine factor IX DNA (Gene Bank Accession Number: NM_001003323). In addition, a TaqMan genotyping assay specific for the detected mutation, was performed in all these dogs. In order to prove the functional relevance of the detected mutation, amino acid alignment studies as well as protein structure modelling analyses were conducted. To investigate, if the mutation is just a single nucleotide polymorphism (SNP) unrelated to haemophilia B, additional TaqMan genotyping assays specific for the mutation were performed in DNA samples of 30 Rhodesian Ridgebacks with undefined haemophilia B status and samples of three other dog breeds (Doberman Pinscher: n = 20; German Wire haired Pointer: n = 20; Labrador: n = 25).

### Animals and sample material

Rhodesian Ridgebacks with known status of haemophilia B were presented as patients or for haemophilia diagnosis to the Small Animal Clinic, University of Veterinary Medicine Hannover. The six phenotypically affected male Rhodesian Ridgebacks had all a known bleeding history and substantially reduced factor IX activities of approximately 1% (Table 2). Clinical signs mainly included severe bleeding after minor surgeries and injuries and occasional spontaneous bleeding. Among the four bitches with suspected carrier status due to moderately reduced factor IX activity (48-69%), one was confirmed by affected littermates. Twelve healthy Rhodesian Ridgeback dogs had a factor IX activity within the reference range and no hint for an excessive bleeding within their history.

### Blood sample collection

Citrated blood for factor IX activity measurements was obtained from a cephalic or saphenous vein using sterile disposable needles (1.1 λ 30 mm) and only slight pressure was used to raise the vein. Citrated blood was collected into plastic tubes containing one part (1 mL) 0.11 mol/L sodium citrate solution for nine parts (9 mL) of blood and immediately mixed by careful rocking. Platelet poor citrated plasma for factor IX measurements was gained by centrifugation twice for 10 min at 2000 g. The final supernatant was then collected and stored in aliquots at -70 °C. In addition, 5 mL of EDTA blood were collected for genetic tests.

### Factor IX activity measurements

Coagulation factor IX activities were automatically measured using a routine coagulometric test, which was optimised for canine sample material and is based on commercial human deficient plasma (Mischke, 2001). Measurements were performed in an AMAX Destiny coagulation analyser (Trinity Biotech). A mixture of 20 µL of diluted citrated plasma (1:40 diluted with imidazole buffer, Siemens Healthcare Diagnostics), 20 µL of factor IX deficient plasma (Siemens Healthcare Diagnostics), and 20 µL activating reagent (PTT reagent^{®}, Diagnostica Stago) was incubated, and exactly after 3 min 20 µL 25 mmol/L CaCl₂ solution (Diagnostica Stago) were added as starting reagent. A calibration curve was prepared with different dilutions of a canine pooled plasma corresponding to 200% (1:20), 150% (1:26.7), 125% (1:32), 100% (1:40), 75% (1:53.3), 50% (1:80), 25% (1:160), 10% (1:400), 5% (1:800), and 1% (1:4000) which were used instead of the diluted sample. Canine pooled plasma was prepared by mixing identical citrated plasma aliquots from 100 clinically healthy dogs with unremarkable haematological and biochemical profile and its factor IX activity was defined as 100%. All results are mean values of measurements in duplicate. The reference range for this test is 75-140%.

### DNA isolation and amplification

Genomic DNA was extracted from 200 µL EDTA whole blood using a commercially available kit Biosprint 15 DNA Blood Kit (Qiagen) following manufacturers' instructions. The canine factor IX cDNA sequence was utilised to search the dog genome database at NCBI and the eight exons of the canine factor IX gene were determined. Nine pairs of oligonucleotide primers (sequences and PCR conditions are shown in Table 4) were used to amplify the coding region and the exon-intron boundaries of the canine factor IX gene using genomic DNA of the dogs. Target sequences were amplified in 50 µL reaction mixtures under standard reaction conditions containing approximately 50 ng genomic DNA and 0.2 µmol/L of each primer.

### DNA sequencing

PCR products were purified prior to sequencing using Min Elute PCR Purification Kit (Qiagen). Sequencing was done by cycle sequencing using DyeDeoxy Terminators (Applied Biosystems) in an automated sequencer ABI 3130 Genetic Analyzer (Applied Biosystems). Sequence comparison was performed between different groups of Rhodesian Ridgebacks with defined haemophilia B status using BLAD ClustalW alignment.

### Rapid screening of the missense mutation

TaqMan genotyping assay specific for the mutation was designed using File Builder Software Version 3.1 (Applied Biosystems). The region flanking the SNP was amplified in the presence of two allele-specific fluorescent probes. One probe labelled with VIC dye, detected the wild type allele sequence and one probe labelled with FAM dye detected the mutant allele sequence. PCR amplification and allelic discrimination was performed using Rotor-Gene 6000 (Corbett).

### Amino acid sequence alignment and domain analysis

Alignment of the primary structure of the protease domain of canine factor IX with those of human factor IX, bovine trypsin, bovine chymotrypsin, porcine elastase and porcine kallikrein was based on sequence identity and structural topological equivalence and used the programme Clustal (Larkin et al., 2007). Domain analysis applied SMART (Letunic et al., 2009) and ProSite (Sigrist et al., 2009), detailed methods have been described previously (Gaudermann et al., 2006).

### Structural protein modelling

Structural protein modelling applied CPHmodels (Lund et al., 2002) to generate a structure model and used PyMol (DeLano, 2002) for graphical analysis.

### Results

### Identification and rapid screening of the missense mutation

Comparison of the entire coding region of the canine factor IX DNA sequence and of the exon-intron junctions from six haemophilic dogs with severe factor IX deficiency (Nos. 1-6) with the wild type canine factor IX DNA revealed a G-A missense mutation at nucleotide 752 in exon 7 of the haemophilic factor IX gene. This mutation results in a glycine (GGA) to glutamic acid (GAA) exchange in the catalytic domain of the haemophilic factor IX. All affected dogs were hemizygous for the detected mutation in exon 7 (Table 2). Four dogs (Nos. 7-10), which were suspected or proven carriers, were found to be heterozygous for the G-A mutation and none of the healthy Rhodesian Ridgebacks was affected by the mutation. No further alterations in the sequences between affected dogs and the healthy control group could be observed. In all cases of Rhodesian Ridgebacks with defined haemophilia B status, the result of TaqMan genotyping assay confirmed the haemophilia B status and thereby the results of the sequencing analysis. Analyses with TaqMan genotyping assay did not reveal the presence of the G-A missense mutation in exon 7 of canine factor IX DNA in 30 further Rhodesian Ridgebacks with undefined haemophilia B status and 65 animals of three other dog breeds.

### Amino acid sequence alignment and domain analysis

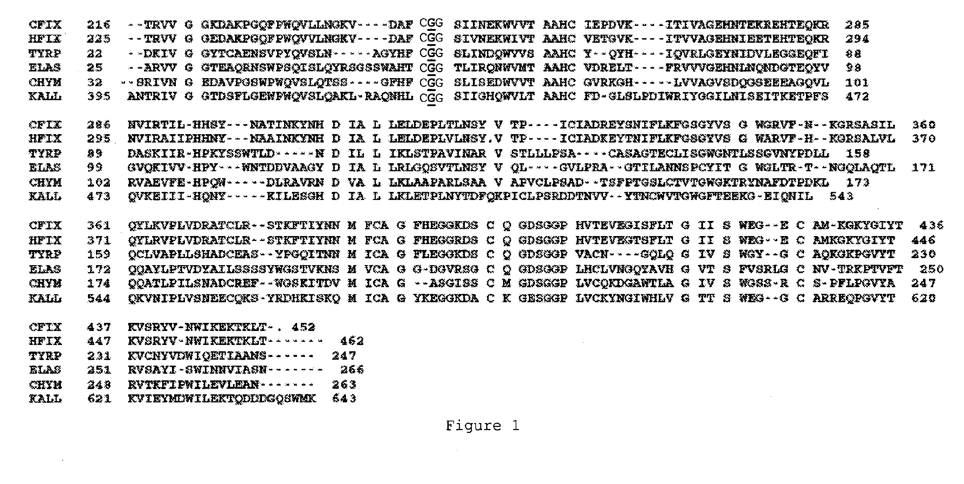

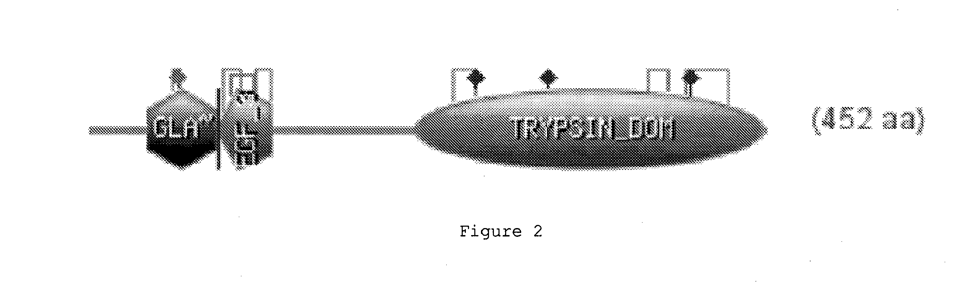

The catalytic domain of trypsin-like serine proteases is highly conserved throughout evolution. Amino acid sequence alignment of the members of this family of proteases demonstrates that certain amino acids are homologous between mammals. Fig. 1 shows the amino acid sequence alignment of the heavy chain of canine factor IX with the corresponding region of several other serine proteases (human factor IX, bovine trypsin, porcine elastase, bovine chymotrypsin, porcine kallikrein). Fig. 1 demonstrates that the mutation in canine factor IX affects one of the highly conserved amino acids, substituting a negatively charged residue (glutamic acid) for an amino acid that is uncharged and has no side chain (glycine). Domain analysis shows, that mutation of amino acid 244 from G to E is located in the trypsin domain of factor IX (Fig. 2). It also is directly next to a disulfide bridge (residues 243 and 259).

### Structural protein modelling

Structure analysis shows that a change in conformation is most likely occurring within the inner part of the protein located close to the active centre due to the fact that the mutation to glutamic acid leads to a long side chain that needs more space and disturbs the structure even more due to its charge (Figs. 3 and 4). The mutation of residue 244 leads to a change in the structure of the pocket-shaped active site of the trypsin domain. The structural proximity is evident: The mutation lies directly adjacent to a disulfide bridge stabilizing the active site and causes, due to the changed structure of the mutated amino acid, a change in the geometry of the disulfide bridge and thus also in the active site.

### Discussion

The results of the present study reveal with sufficient certainty that the detected G-A missense mutation in exon 7 is the responsible mutation for severe haemophilia B in Rhodesian Ridgebacks. The presence of the mutation was confirmed by two different techniques in 22 dogs with defined haemophilia B status. In addition, a cohort of samples from different breeds did not show the observed mutation. The latter results suggest strongly that the detected mutation cannot be a common and non-important mutation.

Amino acid sequence alignment and protein structural modelling analysis demonstrates that in haemophilic Rhodesian Ridgebacks a non-conserved amino acid substitution caused by the mutation leads to a major change in structure and activity: Based on the structure analysis of the protein created by protein modelling, the detected mutation most likely results in an activity reduction of factor IX, well in accordance with the low residual factor IX activity assessed in the functional coagulometric test of approximately 1% and the correspondent severe clinical signs. This further supports the hypothesis that the detected mutation is responsible for the haemophilia B in Rhodesian Ridgebacks. In addition, a similar change in the canine DNA resulted in a similar clinical condition (Evans et al., 1989; Table 1).

Factor IX activities measured in the haemophilic dogs were lower than in the previous report on one haemophilic Rhodesian Ridgeback (4%) (Lutze et al., 2000). Based on clinical criteria applied to humans, the residual factor IX activity corresponds to a severe (<1%) or moderately severe (1-5%) haemophilia severity degree (Rizza, 1977) and, in general, dogs seem to have a more severe clinic at a defined degree of factor activity reduction due to their discrepant behaviour (Mischke et al., 1996; Lutze et al., 2000). In addition, the major structure abnormality of the factor IX molecule is well in accordance with the fact that a heterologous antibody against the human factor IX, which cross-reacted with the normal canine factor IX molecule, showed reduced affinity to bind at the respective domain in the defect factor IX molecule in affected Rhodesian Ridgebacks (Lutze et al., 2000). In the cited study, the haemophilic Rhodesian Ridgeback dog (factor IX activity: 4%) had an antigen concentration of 25% and a carrier (factor IX activity: 56%) an antigen concentration of 53% of a canine pool plasma. The fact that an ELISA with a heterologous antibody was used in this study may have enhanced this phenomenon.

Without a known genetic background, diagnosis of female carriers of a defect is difficult. Apart from analyses on littermates it is mainly based on factor measurements. The present study demonstrates that factor IX activity in carriers confirmed by genetic analyses (maximum value 69%) can nearly reach the lower limit of the reference range (75%). It is therefore likely that under less optimal conditions (e.g., sample shipment to the laboratory, less optimally calibrated test), carrier detection based on factor IX activity measurements is unreliable. The developed TaqMan genotyping assay specific for the detected mutation is therefore a valuable tool for reliable detection of carriers, which is essential for effective breeding hygiene programmes. Unless the fact, that none of the 30 unselected Rhodesian Ridgebacks was tested positive for the mutation, investigation of larger numbers of dogs of this breed seems valuable to define the actual prevalence of the disease.

### Conclusions

A G-A missense mutation in exon 7 of the canine factor IX gene was identified as the likely mutation responsible for severe haemophilia B in Rhodesian Ridgebacks. This mutation results in a glycine (GGA) to glutamic acid (GAA) exchange in the catalytic domain of the haemophilic factor IX.

While the present inventions have been described and illustrated in conjunction with a number of specific embodiments, those skilled in the art will appreciate that variations and modifications may be made without departing from the principles of the inventions as herein illustrated, as claimed. The described embodiments are considered in all respects to be illustrative and not restrictive. The scope of the inventions are, therefore, indicated by the appended claims, rather than by the foregoing description. All changes which come within the meaning and range of equivalence of the claims are to be embraced within their scope.

### References Cited

Brooks, M.B., Gu, W., Ray, K., 1997. Complete deletion of the factor IX gene and inhibition of factor IX activity in a Labrador retriever with hemophilia B. Journal of American Veterinary Medical Association 211, 1418-1421.
DeLano, W.L., 2002. The PyMOL Molecular Graphics System. DeLano Scientific, Palo 277 Alto, CA, USA.
Evans, J.P., Brinkhous, K.M., Brayer, G.D., Reisner, H.M., High, K.A., 1989. Canine hemophilia B resulting from a point mutation with unusual consequences. Proceedings of the National Academy of Sciences USA 86, 10095-10099.
Gaudermann, P., Vogl, I., Zientz, E., Silva, F.J., Moya, A., Gross, R., Dandekar, T., 2006. Analysis of and function predictions for previously conserved hypothetical or putative proteins in Blochmannia floridanus. BMC Microbiology 6, 1-10.
Gu, W., Brooks, M., Catalfamo, J., Ray, J., Ray, K., 1999. Two distinct mutations cause severe hemophilia B in two related canine pedigrees. Thrombosis Haemostasis 82, 1270-1275.
Larkin, M.A., Blackshields, G., Brown, N.P., Chenna, R., McGettigan, P.A., McWilliam, H., Valentin, F., Wallace, I.M., Wilm, A., Lopez, R., Thompson, J.D., Gibson, T.J., Higgins, D.G., 2007. Clustal W and Clustal X version 2.0. Bioinformatics 23, 2947-2948.
Letunic, I., Doerks, T., Bork, P., 2009. SMART 6: recent updates and new developments. Nucleic Acids Research 37, D229-D232.
Lund, O., Nielsen, M., Lundegaard, C., Worning, P., 2002. CPHmodels 2.0: X3M a computer program to extract 3D models. In: CASP5: Proceedings of the 5th Meeting on the Critical Assessment of Techniques for Protein Structure Prediction. 1-5 December 2002, Asilomar, California, USA, Abstract A102.
Lutze, G., Kutschmann, K., Lutze Jr., G., Lichtenfeld, W., 2000. Diagnostic aspects of haemophilia B in dogs. Tierärztliche Praxis 28, 369-373 (in German).
Mauser, A.E., Whitlark, J., Whitney, K.M., Lothrop, C.D., 1996. A deletion mutation causes hemophilia B in Lhasa Apso dogs. Blood 88, 3451-3455.
Mischke, R., 2001. Optimization of coagulometric tests that incorporate human plasma for determination of coagulation factor activities in canine plasma. American Journal of Veterinary Research 62, 625-629.
Mischke, R., Rivera Ramirez, P.A., Deniz, A., Hänies, R., Otto, K., 1996. Haemophilia A in the dog: symptoms, blood coagulation analysis and therapy. Berliner und Münchener Tierärztliche Wochenschrift 109, 279-287.
Rizza, C.R., 1977. Clinical management of haemophilia. British Medical Bulletin 33, 225-230.
Sigrist, C.J., Cerutti, L., de Castro, E., Langendijk-Genevaux, P.S., Bulliard, V., Bairoch, A., Hulo, N., 2009. PROSITE, a protein domain database for functional characterization and annotation. Nucleic Acids Research (Epub).

**Table 1**

| Breed | Mutation in canine factor IX gene | References |
|---|---|---|
| Mixed-breed, originally Cairn terrier and Beagle [Chapel IIill Colony) | G → A transition at nucleotide 1477 in the region encoding the catalytic domain (substitution of glutamic acid for glycine) | Evans et al. (1989) |
| Lhasa Apso | Deletion including nucleotides 772-776 and a C → T transition at nucleotide 777 | Mauser et al. (1996) |
| Labrador | Complete deletion of factor IX gene | Brooks et al. (1997) |
| Pit Bull Terrier | Large deletion mutation spanning the entire 5' region of the factor IX gene extending to exon 6 | Gu et al (1999) |
| Airedale Terrier | 5 kb insertion in exon 8, which with exon 7 encodes the factor IX catalytic region | Gu et al. (1999) |

**Table 2**

| Dog No. | Internal number | Signalement, pedigree^{a} | Clinical signs^{b} | Factor IX activity | Sequence analysis^{c} | TaqMan^{c} |
|---|---|---|---|---|---|---|
| 1 | P 1549 | Male, 3 months | - | 1 | a | a |
| 2 | P 1551 | Male, 2 years. 5 months | - | <1 | a | a |
| 3 | P 1554 | Male, 1 year, 2 months | - | 3 | a | a |
| 4 | P 1615 | Male, 6 months (brother of No. 4, son of No. 10) | - | 1 | a | a |
| 5 | P 1621 | Male, 6 month (brother of No. 4, son of No. 10) | - | 1 | a | a |
| 6 | P 1867 | Male, 2 years, 8 months | - | 1 | a | a |
| 7 | N 983 | Female, 2 years, 2 months | - | 48 | c | c |
| 8 | P 1520 | Female, 2 years, 7 months | - | 62 | c | c |
| 9 | P 1552 | Female, 5 months | - | 69 | c | c |
| 10 | P 1616 | Female, 3 years, 2 months | - | 65 | c | c |
| 11 | N 909 | Female, 1 year, 7 months | - | 84 | n | n |
| 12 | N 910 | Female, 1 year, 6 months | - | 92 | n | n |
| 13 | N 988 | Male, 3 years, 4 months | - | 104 | n | n |
| 14 | N 989 | Male, 5 years, 1 month | - | 100 | n | n |
| 15 | 156834 | Male, 7 years, 11 months | - | 154 | n | n |
| 16 | 156854 | Female, 2 months | - | 109 | n | n |
| 17 | P 1519 | Female, 4 months | | 77 | n | n |
| 18 | 105960 | Male, 2 years, 2 months | - | 127 | n | n |
| 19 | Txxx | Male castr., 2 years, 6 months | - | 110 | n | n |
| 20 | P 1943 | Male, 1 year, 1 month | - | 87 | n | n |
| 21 | 158120 | Male castr., 1 year, 9 months | - | 98 | n | n |
| 22 | 148756 | Male, 7 years, 4 months | - | 150 | n | n |

| | | | | | | |
|---|---|---|---|---|---|---|
| ^{a} Confirmed carrier, mother of Nos. 4 and 5. ^{b} Severe bleeding after minor surgeries and injuries, occasional spontaneous bleeding. ^{c} n = Normal, c = carrier, a = affected. | | | | | | |

**Table 3**

| ProSite predicted features for trypsin domain: |
|---|
| DISULFID 243 259 By similarity [condition: C-x*-C] |
| ACT_SITE 258 Charge relay system (By similarity) [condition: H] [group: 1] |
| ACT_SITE 306 Charge relay system (By similarity) [condition: D] [group: 1] |
| DISULFID 373 387 By similarity [condition: C-x*-C] |
| DISULFID 398 426 By similarity [condition: C-x*-C] |
| ACT_SITE 402 Charge relay system (By similarity) [condition: S] [group: 1] |
| Absent feature: DISULFID 342 408 By similarity [condition not true: C-x*-C] |

**Table 4**

| **Primer** | **Seq-ID No.** | **Sequence (5' → 3')** | **Binding** | **Annealing Temperature** |
|---|---|---|---|---|
| cFIXg-for-1 | 4 | TGTGTCACTTCCGGCTTCAG | Intron 1 | 58°C |
| cFIXg-rev-1 | 5 | TCTACAGCTAGAAGACAAGCATAC | Intron 2 | |
| cFIXg-for-2 | 6 | GATAAATTGGCTTTGGGATTACTTGG | Intron 2 | 58°C |
| cFIXg-rev-2 | 7 | GTACTTTGCATCTGAAGAACATTACG | Intron 3 | |
| cFIXg-for-3 | 8 | GCAGTTTTGAAGAAGCACGGG | Exon 2 | 58°C |
| cFIXg-rev-3 | 9 | ACACAGAGAAAAGATACCTAATTCTCA | Intron 4 | |
| cFIXg-for-4 | 10 | AAGACAGGGGCATCCCATAATC | Intron 4 | 58°C |
| cFIXg-rev-4 | 11 | CTTCCATTAAGTGTTCCTTACCAC | Intron 5 | |
| cFIXg-for-5 | 12 | CTCCCAAGCCTCTTTCCATG | Intron 5 | 58°C |
| cFIXg-rev-5 | 13 | CAAACAGGGTTTATGAAAGTATGTGAAC | Intron 6 | |
| cfix-g-6-for | 14 | CCGCTTGCCAATGAAAAATA | Intron 6 | 58°C |
| cfix-g-6-rev | 15 | CTCTGGGCTCCAGTTTTGAC | Intron 7 | |
| cFIXg-for-7 | 16 | ACACCCCTGCCTATCAACAG | Intron 7 | 58°C |
| cFIXg-rev-7 | 17 | GCTCCTCTAGCATTAGCCC | Intron 8 | |
| cFIXg-for-8 | 18 | CCTTGGCAAATACGTTTATGTGTAAG | Intron 8 | 58°C |
| cFIXg-rev-8 | 19 | TCATGGAAGCCAGCACAGAAC | Exon 8 | |
| cfix-g-9-for | 20 | TTCCTCAAATTTGGGTCTGG | Exon 8 | 58°C |
| cfix-g-9-rev | 21 | CCTAAACGTGTCAACCTTGGA | Exon 8 | |

## Claims

1. Isolated Canine factor IX gene according to Seq-ID No. 1 having a point mutation, wherein nucleotide 752 is A instead of G.

2. Method for the analysis of a genotype of a dog in respect of haemophilia B by a genetic test for identification of at least one point mutation in Seq-ID No. 1 of the genome of the dog, wherein at least one point mutation leads to an amino acid exchange in position 244 of canine factor IX, wherein one point mutation is a point mutation in nucleotide 752 and wherein the point mutation in nucleotide 752 is a point mutation, where nucleotide 752 is A instead of G, and wherein at least this point mutation defines the genotype of the dog in respect of haemophilia B.

3. Method according to claim 2, wherein PCR is used for amplification of a region of Seq-ID No. 1, which contains the point mutation of nucleotide 752.

4. Method according to one of the claims 2 or 3, wherein the dog is a Rhodesian Ridgeback.

5. Process for the determination of the genetic disposition for haemophilia B of a dog, **characterized by** the use of the canine factor IX gene of the dog according to Seq-ID No. 1 having at least one point mutation in Seq-ID No. 1 of the genome of the dog, wherein at least one point mutation leads to an amino acid exchange in position 244 of canine factor IX, and wherein one point mutation is a point mutation, wherein nucleotide 752 is A instead of G, and wherein at least this point mutation determines the genetic disposition for haemophilia B of the dog.

6. Use of the process according to claim 5 for the selection of a Rhodesian Ridgeback for breeding.

## Patentansprüche

1. Isoliertes canines Faktor-IX-Gen nach Seq-ID No. 1, das eine Punktmutation aufweist, wobei das Nukleotid 752 A anstelle von G ist.

2. Verfahren zum Analysieren eines Genotyps eines Hundes in Bezug auf Hämophilie B mittels eines Gentests zum Identifizieren von zumindest einer Punktmutation in Seq-ID No. 1 des Genoms des Hundes, wobei zumindest eine Punktmutation zu einem Aminosäurenaustausch an Position 244 des caninen Faktors IX führt, wobei es sich bei einer Punktmutation um eine Punktmutation in Nukleotid 752 handelt und wobei die Punktmutation in Nukleotid 752 eine Punktmutation ist, bei der Nukleotid 752 A anstelle von G ist, und wobei zumindest diese Punktmutation den Genotyp des Hundes in Bezug auf Hämophilie B definiert.

3. Verfahren nach Anspruch 2,
wobei PCR angewendet wird, um einen Abschnitt der Seq-ID No. 1, welcher die Punktmutation des Nukleotids 752 enthält, zu amplifizieren.

4. Verfahren nach einem der Ansprüche 2 oder 3,
wobei der Hund ein Rhodesian Ridgeback ist.

5. Verfahren zum Feststellen einer genetischen Disposition eines Hundes für Hämophilie B,
**gekennzeichnet durch**
das Verwenden des caninen Faktor-IX-Gens des Hundes nach Seq-ID No. 1, das zumindest eine Punktmutation in Seq-ID No. 1 des Genoms des Hundes aufweist, wobei zumindest eine Punktmutation zu einem Aminosäurenaustausch an Position 244 des caninen Faktors IX führt, und wobei es sich bei einer Punktmutation um eine Punktmutation handelt, bei der das Nukleotid 752 A anstelle von G ist, und wobei zumindest diese Punktmutation die genetische Disposition des Hundes für Hämophilie B bestimmt.

6. Anwendung des Verfahrens nach Anspruch 5 zur Auswahl eines Rhodesian Ridgebacks für die Zucht.

## Revendications

1. Gène canin isolé pour le facteur IX selon Seq-ID No. 1 ayant une mutation ponctuelle dans laquelle le nucléotide 752 est A au lieu de G.

2. Procédé pour l'analyse d'un génotype d'un chien en relation à l'hémophilie B par un test génétique pour l'identification d'au moins une mutation ponctuelle dans Seq-ID No. 1 du génome du chien, dans lequel au moins une mutation ponctuelle donne lieu à un échange d'acides aminés en position 244 du facteur IX canin, une mutation ponctuelle étant une mutation ponctuelle dans le nucléotide 752 et la mutation ponctuelle dans le nucléotide 752 étant une mutation ponctuelle dans laquelle le nucléotide 752 est A au lieu de G, et au moins cette mutation ponctuelle définissant le génotype du chien en relation à l'hémophilie B.

3. Procédé selon la revendication 2,
dans lequel la PCR est utilisée pour l'amplification d'une région de Seq-ID No. 1 qui comporte la mutation ponctuelle du nucléotide 752.

4. Procédé selon l'une quelconque des revendications 2 ou 3,
dans lequel le chien est un chien de Rhodésie.

5. Procédé pour la détermination de la prédisposition génétique pour l'hémophilie B d'un chien,
**caractérisé par**
l'utilisation du gène canin pour le facteur IX du chien selon Seq-ID No. 1 ayant au moins une mutation ponctuelle dans Seq-ID No. 1 du génome du chien, au moins une mutation ponctuelle donnant lieu à un échange d'acides aminés en position 244 du facteur IX canin, et une mutation ponctuelle étant une mutation ponctuelle dans laquelle le nucléotide 752 est A au lieu de G, et au moins cette mutation ponctuelle déterminant la prédisposition génétique pour l'hémophilie B du chien.

6. Utilisation du procédé selon la revendication 5 pour la sélection d'un chien de Rhodésie pour la reproduction.


### SEQUENCE LISTING

<110> LABOKLIN GmbH & Co. KG
<120> Genetic test for genetic disposition of haemophilia B in Rhodesian Ridgeback
<130> W/LAO-006-EP-2
<160> 21
<170> PatentIn version 3.5
<210> 1
   <211> 3080
   <212> DNA
   <213> Canis familiaris
<220>
   <221> gene
   <222> (1)..(3080)
   <223> F9
   coagulation factor IX
GeneID:404015
<220>
   <221> CDS
   <222> (22)..(1380)
   <223> /protein_id="NP_001003323.1" /db_xref="GI:50979168"
   /db_xref="GeneID:404015"
<220>
   <221> mutation
   <222> (752)..(752)
   <223> G= wild type; A= haemophilia B
<400> 1
   aagcagcgcc tgaataggat c atg gca gaa gca tcg ggc ctc gtc acc gtc        51
                           Met Ala Glu Ala Ser Gly Leu Val Thr Val
                           1               5                   10
   tgc ctt tta gga tat cta ctc agt gcc gaa tgt gca gtt ttt ctt gat        99
   Cys Leu Leu Gly Tyr Leu Leu Ser Ala Glu Cys Ala Val Phe Leu Asp
                   15                  20                  25
   cgt gaa aat gcc acc aaa att ctg agt cgg cca aag agg tat aat tca       147
   Arg Glu Asn Ala Thr Lys Ile Leu Ser Arg Pro Lys Arg Tyr Asn Ser
               30                  35                  40
   ggt aaa ctg gaa gag ttt gtt cga ggg aac ctt gag aga gaa tgt ata       195
   Gly Lys Leu Glu Glu Phe Val Arg Gly Asn Leu Glu Arg Glu Cys Ile
           45                  50                  55
   gaa gaa aag tgc agt ttt gaa gaa gca cgg gaa gtt ttt gaa aac act       243
   Glu Glu Lys Cys Ser Phe Glu Glu Ala Arg Glu Val Phe Glu Asn Thr
       60                  65                  70
   gaa aaa acc act gaa ttt tgg aag caa tat gtt gat gga gat caa tgt       291
   Glu Lys Thr Thr Glu Phe Trp Lys Gln Tyr Val Asp Gly Asp Gln Cys
   75                  80                  85                  90
   gaa tcc aat cca tgt tta aat gac ggt gta tgc aag gat gac att aat       339
   Glu Ser Asn Pro Cys Leu Asn Asp Gly Val Cys Lys Asp Asp Ile Asn
                       95                  100                 105
   tcc tat gaa tgt tgg tgt cga gct gga ttt gaa gga aag aac tgt gaa      387
   Ser Tyr Glu Cys Trp Cys Arg Ala Gly Phe Glu Gly Lys Asn Cys Glu
               110                 115                 120
   tta gat gta aca tgc aac att aag aat ggc aga tgc aag cag ttt tgt      435
   Leu Asp Val Thr Cys Asn Ile Lys Asn Gly Arg Cys Lys Gln Phe Cys
           125                 130                 135
   aaa ttg ggc ccc gat aac aag gtg gtt tgt tcc tgt act acg gga tac      483
   Lys Leu Gly Pro Asp Asn Lys Val Val Cys Ser Cys Thr Thr Gly Tyr
       140                 145                 150
   caa ctt gcg gaa gac caa agg tcc tgt gaa cca gca gtg cca ttt cca      531
   Gln Leu Ala Glu Asp Gln Arg Ser Cys Glu Pro Ala Val Pro Phe Pro
   155                 160                 165                 170
   tgt gga aga gtt tct gtc cct cac att tct atg aca cgc acc cgt gct      579
   Cys Gly Arg Val Ser Val Pro His Ile Ser Met Thr Arg Thr Arg Ala
                   175                 180                 185
   gaa act ctt ttt tcc aat atg gac tat gaa aat tca act gaa gtg gaa      627
   Glu Thr Leu Phe Ser Asn Met Asp Tyr Glu Asn Ser Thr Glu Val Glu
               190                 195                 200
   aaa att ttg gat aac gtc acc caa ccg ctt aac gac ttc act cga gtt      675
   Lys Ile Leu Asp Asn Val Thr Gln Pro Leu Asn Asp Phe Thr Arg Val
           205                 210                 215
   gtt ggt gga aaa gat gcc aaa cca ggt caa ttc cct tgg cag gtc ctt      723
   Val Gly Gly Lys Asp Ala Lys Pro Gly Gln Phe Pro Trp Gln Val Leu
       220                 225                 230
   ttg aat ggg aaa gtt gat gca ttc tgc gga ggt tcc atc atc aat gaa      771
   Leu Asn Gly Lys Val Asp Ala Phe Cys Gly Gly Ser Ile Ile Asn Glu
   235                 240                 245                 250
   aaa tgg gtg gta act gca gcc cac tgt att gag cct gat gtt aaa att      819
   Lys Trp Val Val Thr Ala Ala His Cys Ile Glu Pro Asp Val Lys Ile
                   255                 260                 265
   acc ata gtt gca ggt gag cat aac acc gag aag agg gaa cat aca gag      867
   Thr Ile Val Ala Gly Glu His Asn Thr Glu Lys Arg Glu His Thr Glu
               270                 275                 280
   cag aag cga aac gtg att cgc act att ctt cac cac agc tat aat gca      915
   Gln Lys Arg Asn Val Ile Arg Thr Ile Leu His His Ser Tyr Asn Ala
           285                 290                 295
   act att aat aag tac aac cat gac atc gcc ctt ctg gaa ctg gat gag      963
   Thr Ile Asn Lys Tyr Asn His Asp Ile Ala Leu Leu Glu Leu Asp Glu
       300                 305                 310
   ccc tta acg ctg aac agc tat gta aca cct att tgc att gct gac agg     1011
   Pro Leu Thr Leu Asn Ser Tyr Val Thr Pro Ile Cys Ile Ala Asp Arg
   315                 320                 325                 330
   gaa tac tcg aac atc ttc ctc aaa ttt ggg tct ggc tat gtg agt ggc     1059
   Glu Tyr Ser Asn Ile Phe Leu Lys Phe Gly Ser Gly Tyr Val Ser Gly
                   335                 340                 345
   tgg ggg aga gtc ttc aac aaa ggg cga tcg gct tca att ctt caa tac     1107
   Trp Gly Arg Val Phe Asn Lys Gly Arg Ser Ala Ser Ile Leu Gln Tyr
               350                 355                 360
   ctt aaa gtt cca ctt gtt gac cga gcc acg tgc ctt cgg tcc acg aag     1155
   Leu Lys Val Pro Leu Val Asp Arg Ala Thr Cys Leu Arg Ser Thr Lys
           365                 370                 375
   ttc acc att tat aac aac atg ttc tgt gct ggc ttc cat gag gga ggt      1203
   Phe Thr Ile Tyr Asn Asn Met Phe Cys Ala Gly Phe His Glu Gly Gly
       380                 385                 390
   aaa gat tca tgc cag ggc gat agt ggg gga ccc cat gtc acc gaa gta      1251
   Lys Asp Ser Cys Gln Gly Asp Ser Gly Gly Pro His Val Thr Glu Val
   395                 400                 405                 410
   gaa ggc ata agt ttc tta act ggg att att agc tgg ggt gaa gag tgt      1299
   Glu Gly Ile Ser Phe Leu Thr Gly Ile Ile Ser Trp Gly Glu Glu Cys
                   415                 420                 425
   gcg atg aaa ggg aag tat gga ata tat acc aag gtg tcc cgg tat gtc      1347
   Ala Met Lys Gly Lys Tyr Gly Ile Tyr Thr Lys Val Ser Arg Tyr Val
               430                 435                 440
   aac tgg att aaa gaa aag acg aag ctc acc taa agaataatgt atttccaagg    1400
   Asn Trp Ile Lys Glu Lys Thr Lys Leu Thr
           445                 450
   ttgacacgtt tagggtagaa aatggacaag gtcctttact aactaatcac tttttttatc    1460
   tctttagatt tgcactatat acattctctg catactgctt tttctctttc tggggagaaa    1520
   tctatctaga attcctattt tactagacta agtgaattag aaaatgtaat cactacggga    1580
   atgtactgtg atgggacttg tgaccactcc cacaggtcta gcccttggca ccattgtgag    1640
   gttaggttat tcctccgtcc cgtcagtgtt aagtttctcc actggggcaa ctccctgatt    1700
   ctccctcctt ggcagcattc catgttccag acctttctta cctttcccat ggaaagcatc    1760
   atcaaaatgt gttagatcta catccaggat atttgatcta gttcagaaca agtctaacac    1820
   ctcacttgtt aaggaagaac acaggagcaa ctgacaggtt gcaactcacc agaaaacact    1880
   atttcctttt ctgtatgctt attcctgctt ccttatctct tctgtttcct aatcctgaaa    1940
   tcagtgtctc tctttctctt tctctctctc tttttcctac agaggtttaa aggagggaag    2000
   ggacacatca tgctgtttta ctactgtcca cagttataca tgtctatcaa acccagactt    2060
   gctttcagtt tggtctttga cttgcttttc ggagcatagg gatgaagcca ggtgcctgaa    2120
   gaccttcagg aaaatttgtc tgaaagagtc atgttactga aatacatggc atcgtggaag    2180
   gaatgaccga cccatcagaa tacgtccaat aagcatttgt aattgtgttg tgatagaggt    2240
   aactaagaag agtgacatca actcctgcgt ctcatccccc atgtgaaaaa actgtgaact    2300
   aaaagagaac agccagtgtg caacgtagaa ctagtagagt cttaaggaag aattcattgg    2360
   tgggtctcca gtactggccg gagctaagga agaagttgcc ctcgaccaaa gaacatgagc    2420
   accctgtctc ttaaactagc atgtccccac agtggagaag gatgtactgg tggcttaaag    2480
   gcatgagtca ggggtgtctg ggtggctcag tggttgagcc tttggctcag ggcatgatcc    2540
   cagcgtcctg ggattgagtc ccgcatcagg cttcctgcag ggggcctgct tctccctctg    2600
   cttatgtctc tgcttctctg tgtgtgtgtg tgtgtctctc atgaaaaaaa taaataaaat    2660
   ctttaaaaaa aaaaaaggta tgagtcattc cagtgagcca tctcactccc ccttttctgg    2720
   tttcaggttc tctaggcaac cttttgatta taattaggcc ttccatattg aattttctaa    2780
   agagttgctg accaacctct gcatgtactt tagtgtgtta ggctggctcc ctttatgaat    2840
   taataaactg ttgttctggt tgatactgta gctttttgtg aatgtggctg atgtgaatca 2900
   gtcatcccac atgttgttat atatgagact actgacaaaa tcatgtttgg gaccactttg 2960
   acctgaccaa gctgctgcct tcacctcacc cccagccagg catttacttt agtccttcaa 3020
   atattaagta ccaataaatg tcttttcaaa tttcaaaaaa aaaaaaaaaa aaaaaaaaaa 3080
<210> 2
   <211> 452
   <212> PRT
   <213> Canis familiaris
<400> 2
   Met Ala Glu Ala Ser Gly Leu Val Thr Val Cys Leu Leu Gly Tyr Leu
   1 5 10 15
   Leu Ser Ala Glu Cys Ala Val Phe Leu Asp Arg Glu Asn Ala Thr Lys
   20 25 30
   Ile Leu Ser Arg Pro Lys Arg Tyr Asn Ser Gly Lys Leu Glu Glu Phe
   35 40 45
   Val Arg Gly Asn Leu Glu Arg Glu Cys Ile Glu Glu Lys Cys Ser Phe
   50 55 60
   Glu Glu Ala Arg Glu Val Phe Glu Asn Thr Glu Lys Thr Thr Glu Phe
   65 70 75 80
   Trp Lys Gln Tyr Val Asp Gly Asp Gln Cys Glu Ser Asn Pro Cys Leu
   85 90 95
   Asn Asp Gly Val Cys Lys Asp Asp Ile Asn Ser Tyr Glu Cys Trp Cys
   100 105 110
   Arg Ala Gly Phe Glu Gly Lys Asn Cys Glu Leu Asp Val Thr Cys Asn
   115 120 125
   Ile Lys Asn Gly Arg Cys Lys Gln Phe Cys Lys Leu Gly Pro Asp Asn
   130 135 140
   Lys Val Val Cys Ser Cys Thr Thr Gly Tyr Gln Leu Ala Glu Asp Gln
   145 150 155 160
   Arg Ser Cys Glu Pro Ala Val Pro Phe Pro Cys Gly Arg Val Ser Val
   165 170 175
   Pro His Ile Ser Met Thr Arg Thr Arg Ala Glu Thr Leu Phe Ser Asn
   180 185 190
   Met Asp Tyr Glu Asn Ser Thr Glu Val Glu Lys Ile Leu Asp Asn Val
   195 200 205
   Thr Gln Pro Leu Asn Asp Phe Thr Arg Val Val Gly Gly Lys Asp Ala
           210                 215                 220
   Lys Pro Gly Gln Phe Pro Trp Gln Val Leu Leu Asn Gly Lys Val Asp
   225                 230                 235                 240
   Ala Phe Cys Gly Gly Ser Ile Ile Asn Glu Lys Trp Val Val Thr Ala
                   245                 250                 255
   Ala His Cys Ile Glu Pro Asp Val Lys Ile Thr Ile Val Ala Gly Glu
               260                 265                 270
   His Asn Thr Glu Lys Arg Glu His Thr Glu Gln Lys Arg Asn Val Ile
           275                 280                 285
   Arg Thr Ile Leu His His Ser Tyr Asn Ala Thr Ile Asn Lys Tyr Asn
       290                 295                 300
   His Asp Ile Ala Leu Leu Glu Leu Asp Glu Pro Leu Thr Leu Asn Ser
   305                 310                 315                 320
   Tyr Val Thr Pro Ile Cys Ile Ala Asp Arg Glu Tyr Ser Asn Ile Phe
                   325                 330                 335
   Leu Lys Phe Gly Ser Gly Tyr Val Ser Gly Trp Gly Arg Val Phe Asn
               340                 345                 350
   Lys Gly Arg Ser Ala Ser Ile Leu Gln Tyr Leu Lys Val Pro Leu Val
           355                 360                 365
   Asp Arg Ala Thr Cys Leu Arg Ser Thr Lys Phe Thr Ile Tyr Asn Asn
       370                 375                 380
   Met Phe Cys Ala Gly Phe His Glu Gly Gly Lys Asp Ser Cys Gln Gly
   385                 390                 395                 400
   Asp Ser Gly Gly Pro His Val Thr Glu Val Glu Gly Ile Ser Phe Leu
                   405                 410                 415
   Thr Gly Ile Ile Ser Trp Gly Glu Glu Cys Ala Met Lys Gly Lys Tyr
               420                 425                 430
   Gly Ile Tyr Thr Lys Val Ser Arg Tyr Val Asn Trp Ile Lys Glu Lys
           435                 440                 445
   Thr Lys Leu Thr
       450
<210> 3
   <211> 452
   <212> PRT
   <213> Canis familiaris
<220>
   <221> mutation
   <222> (244)..(244)
   <223> G= wild type; E = haemophilia B
<400> 3
   Met Ala Glu Ala Ser Gly Leu Val Thr Val Cys Leu Leu Gly Tyr Leu
   1               5                   10                  15
   Leu Ser Ala Glu Cys Ala Val Phe Leu Asp Arg Glu Asn Ala Thr Lys
               20                  25                  30
   Ile Leu Ser Arg Pro Lys Arg Tyr Asn Ser Gly Lys Leu Glu Glu Phe
           35                  40                  45
   Val Arg Gly Asn Leu Glu Arg Glu Cys Ile Glu Glu Lys Cys Ser Phe
       50                  55                  60
   Glu Glu Ala Arg Glu Val Phe Glu Asn Thr Glu Lys Thr Thr Glu Phe
   65                  70                  75                  80
   Trp Lys Gln Tyr Val Asp Gly Asp Gln Cys Glu Ser Asn Pro Cys Leu
                   85                  90                  95
   Asn Asp Gly Val Cys Lys Asp Asp Ile Asn Ser Tyr Glu Cys Trp Cys
               100                 105                 110
   Arg Ala Gly Phe Glu Gly Lys Asn Cys Glu Leu Asp Val Thr Cys Asn
           115                 120                 125
   Ile Lys Asn Gly Arg Cys Lys Gln Phe Cys Lys Leu Gly Pro Asp Asn
       130                 135                 140
   Lys Val Val Cys Ser Cys Thr Thr Gly Tyr Gln Leu Ala Glu Asp Gln
   145                 150                 155                 160
   Arg Ser Cys Glu Pro Ala Val Pro Phe Pro Cys Gly Arg Val Ser Val
                   165                 170                 175
   Pro His Ile Ser Met Thr Arg Thr Arg Ala Glu Thr Leu Phe Ser Asn
               180                 185                 190
   Met Asp Tyr Glu Asn Ser Thr Glu Val Glu Lys Ile Leu Asp Asn Val
           195                 200                 205
   Thr Gln Pro Leu Asn Asp Phe Thr Arg Val Val Gly Gly Lys Asp Ala
       210                 215                 220
   Lys Pro Gly Gln Phe Pro Trp Gln Val Leu Leu Asn Gly Lys Val Asp
   225                 230                 235                 240
   Ala Phe Cys Gly Gly Ser Ile Ile Asn Glu Lys Trp Val Val Thr Ala
                   245                 250                 255
   Ala His Cys Ile Glu Pro Asp Val Lys Ile Thr Ile Val Ala Gly Glu
               260                 265                 270
   His Asn Thr Glu Lys Arg Glu His Thr Glu Gln Lys Arg Asn Val Ile
           275                 280                 285
   Arg Thr Ile Leu His His Ser Tyr Asn Ala Thr Ile Asn Lys Tyr Asn
       290                 295                 300
   His Asp Ile Ala Leu Leu Glu Leu Asp Glu Pro Leu Thr Leu Asn Ser
   305                 310                 315                 320
   Tyr Val Thr Pro Ile Cys Ile Ala Asp Arg Glu Tyr Ser Asn Ile Phe
                   325                 330                 335
   Leu Lys Phe Gly Ser Gly Tyr Val Ser Gly Trp Gly Arg Val Phe Asn
               340                 345                 350
   Lys Gly Arg Ser Ala Ser Ile Leu Gln Tyr Leu Lys Val Pro Leu Val
           355                 360                 365
   Asp Arg Ala Thr Cys Leu Arg Ser Thr Lys Phe Thr Ile Tyr Asn Asn
       370                 375                 380
   Met Phe Cys Ala Gly Phe His Glu Gly Gly Lys Asp Ser Cys Gln Gly
   385                 390                 395                 400
   Asp Ser Gly Gly Pro His Val Thr Glu Val Glu Gly Ile Ser Phe Leu
                   405                 410                 415
   Thr Gly Ile Ile Ser Trp Gly Glu Glu Cys Ala Met Lys Gly Lys Tyr
               420                 425                 430
   Gly Ile Tyr Thr Lys Val Ser Arg Tyr Val Asn Trp Ile Lys Glu Lys
           435                 440                 445
   Thr Lys Leu Thr
       450
<210> 4
   <211> 20
   <212> DNA
   <213> Artificial Sequence
<220>
   <223> Primer cFIxg-for-1
<400> 4
   tgtgtcactt ccggcttcag 20
<210> 5
   <211> 24
   <212> DNA
   <213> Artificial Sequence
<220>
   <223> Primer cFIXg-rev-1
<400> 5
   tctacagcta gaagacaagc atac 24
<210> 6
   <211> 26
   <212> DNA
   <213> Artificial Sequence
<220>
   <223> Primer cFIXg-for-2
<400> 6
   gataaattgg ctttgggatt acttgg 26
<210> 7
   <211> 26
   <212> DNA
   <213> Artificial sequence
<220>
   <223> Primer cFIXg-rev-2
<400> 7
   gtactttgca tctgaagaac attacg 26
<210> 8
   <211> 21
   <212> DNA
   <213> Artificial Sequence
<220>
   <223> Primer cFIXg-for-3
<400> 8
   gcagttttga agaagcacgg g 21
<210> 9
   <211> 27
   <212> DNA
   <213> Artificial Sequence
<220>
   <223> Primer cFIXg-rev-3
<400> 9
   acacagagaa aagataccta attctca 27
<210> 10
   <211> 22
   <212> DNA
   <213> Artificial sequence
<220>
   <223> Primer cFIXg-for-4
<400> 10
   aagacagggg catcccataa tc 22
<210> 11
   <211> 24
   <212> DNA
   <213> Artificial Sequence
<220>
   <223> Primer cFIxg-rev-4
<400> 11
   cttccattaa gtgttcctta ccac 24
<210> 12
   <211> 20
   <212> DNA
   <213> Artificial Sequence
<220>
   <223> Primer cFIxg-for-5
<400> 12
   ctcccaagcc tctttccatg 20
<210> 13
   <211> 28
   <212> DNA
   <213> Artificial Sequence
<220>
   <223> Primer cFIXg-rev-5
<400> 13
   caaacagggt ttatgaaagt atgtgaac 28
<210> 14
   <211> 20
   <212> DNA
   <213> Artificial Sequence
<220>
   <223> Primer cfix-g-6-for
<400> 14
   ccgcttgcca atgaaaaata 20
<210> 15
   <211> 20
   <212> DNA
   <213> Artificial Sequence
<220>
   <223> Primer cfix-g-6-rev
<400> 15
   ctctgggctc cagttttgac 20
<210> 16
   <211> 20
   <212> DNA
   <213> Artificial Sequence
<220>
   <223> Primer cFIXg-for-7
<400> 16
   acacccctgc ctatcaacag 20
<210> 17
   <211> 19
   <212> DNA
   <213> Artificial Sequence
<220>
   <223> Primer cFIxg-rev-7
<400> 17
   gctcctctag cattagccc 19
<210> 18
   <211> 26
   <212> DNA
   <213> Artificial Sequence
<220>
   <223> Primer cFIXg-for-8
<400> 18
   ccttggcaaa tacgtttatg tgtaag 26
<210> 19
   <211> 21
   <212> DNA
   <213> Artificial Sequence
<220>
   <223> Primer cFIxg-rev-8
<400> 19
   tcatggaagc cagcacagaa c 21
<210> 20
   <211> 20
   <212> DNA
   <213> Artificial Sequence
<220>
   <223> Primer cfix-g-9-for
<400> 20
   ttcctcaaat ttgggtctgg 20
<210> 21
   <211> 21
   <212> DNA
   <213> Artificial Sequence
<220>
   <223> Primer cfix-g-9-rev
<400> 21
   cctaaacgtg tcaaccttgg a 21